(19) Europäisches Patentamt
European Patent Office
Office européen des brevets

(11) Publication number: 0 276 030 B1

# (12) EUROPEAN PATENT SPECIFICATION

(45) Date of publication of the patent specification: 17.10.90

(51) Int. Cl.[5]: C07C 43/23, C07C 41/16

(21) Application number: 88200018.5

(22) Date of filing: 08.01.88

(54) **Process for the preparation of o-isopropoxyphenol.**

(30) Priority: 14.01.87 IT 1907387

(43) Date of publication of application: 27.07.88 Bulletin 88/30

(45) Publication of the grant of the patent: 17.10.90 Bulletin 90/42

(84) Designated Contracting States: AT BE CH DE ES FR GB GR LI LU NL SE

(56) References cited:
EP-A- 0 040 400
EP-A- 0 052 314
EP-A- 0 092 102
EP-A- 0 151 392

CHEMICAL ABSTRACTS, vol. 103, no. 21, 25 Nov 1985, page 642, column 2, abstract no. 177900q, Columbus, Ohio, US; R. NEUMANN: "Catalyst poisoning and selectivity constants in polyethylene glycol catalysed phase transfer catalysts"
CHEMICAL ABSTRACTS, vol. 96, no. 25, 21 June 1982, page 659, column 1, abstract no. 216942p, Columbus, Ohio, US; Y. KIMURA et al.: "Poly(ethylene glycols) are extraordinary catalysts in liquid-liquid two-phase dehydrohalogenation"

(73) Proprietor: ENICHEM SYNTHESIS S.p.A., Via Ruggero Settimo 55, I-90139 Palermo(IT)

(72) Inventor: Correale, Mariano, Via Volta 9, I-24040 Bonate Sotto (Bergamo)(IT)
Inventor: Minisci, Francesco, Via Marescalchi 19, I-20133 Milano(IT)
Inventor: Panseri, Pietro, Via Giovanni da Campione 20, I-24100 Bergamo(IT)

(74) Representative: Roggero, Sergio et al, Ing. Barzanò & Zanardo Milano S.p.A. Via Borgonuovo 10, I-20121 Milano(IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the synthesis of 2–(1–methoxy)–phenol, more commonly indicated as o–isopropoxy–phenol, having the formula (I):

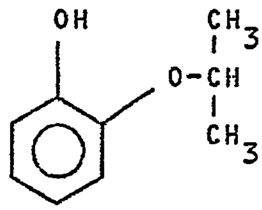

by means of the reaction of 1,2–benzenediol, or pyrocatechol, of formula (II), with an isopropyl halide having formula (III):

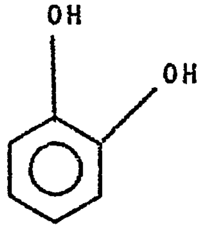

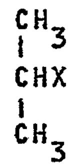

wherein X represents a halogen atom, in the presence of a solid inorganic basis, of a solvent, or of a mixture of inert organic solvents, and of a phase transfer catalyst, characterized in that such a phase transfer catalyst is a polyether.

o-Isopropoxyphenol is a key intermediate in the synthesis of 2–(1–methylethoxy)–phenol methylcarbamate (Propoxur), one from the most widely used insecticides presently available on the market.

Many processes are known from patent literature, and general technical literature, for the synthesis of this intermediate. Among these, a large number of processes relate to the synthesis process by means of the reaction of an alkaline salt of pyrocatechol with an isopropyl halide according to the following reaction diagram:

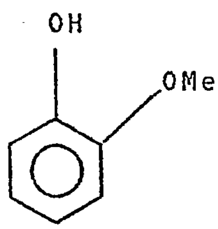

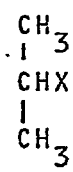

wherein Me represents an alkali metal atom and X is a halogen atom.

However, the mono-etherification according to such reaction diagram shows many drawbacks, already underlined in technical literature, among which the fact that considerable amounts of impurities (substantially ring-alkylating products and 1,2-di-isopropoxy-benzene), and the fact that it is not possible to easily separate and recycle the alkaline salt of pyrocatechol, but, on the contrary, it is necessary to accomplish the complete conversion thereof, which involves a considerable decrease in selectivity, very long, and therefore industrially unacceptable, reaction times, and the use of a large excess of isopropyl halide.

Furthermore, from published European patent application No. 0 151 392 (EP-A-151392), a process is known for the preparation of σ-isopropoxyphenol by means of the reaction of pyrocatechol with an isopropyl halide in the presence of a solid alkaline base and of a phase transfer catalyst selected from quaternary ammonium or phosphonium salts. As stressed by the same European patent application, this reaction is however preferably carried out with isopropyl bromide, because the use of this particular, more reactive, halide, offers, under such reaction conditions as disclosed in said European patent application, the best results. From an industrial standpoint, both as regards the process costs, and as regards the disposal of the reaction byproducts, the use of a bromide is however highly unadvisable.

The present Applicant has found now that by using a polyether as the phase transfer catalyst, extremely good results can be obtained even using as the reaction partner the less reactive, but safer, and much cheaper, isopropyl chloride.

Under the reaction conditions which are disclosed in the following, it is in fact possible to obtain considerably better results, in terms of conversion of pyrocatechol used as the starting material, as compared to those obtained by means of the above referred to European patent application, by using isopropyl chloride in lieu of isopropyl bromide.

The chlorine-containing salt which is obtained as a reaction byproduct, and which will generally be an akali-metal or alkali-earth metal chloride, does not obviously show any disposal problems. Therefore, even if the reaction of the present invention can be carried out by using a whatever isopropyl halide, according to a preferred practical embodiment of the present invention, isopropyl chloride will be used.

The isopropyl halide (III) is generally used in a molar ratio to pyrocatechol (II) comprised within the range of from 0.5 to 2.5, and, preferably, of from 0.8 to 2.

The etherification reaction of the present invention is carried out in the presence of a solid inorganic base, preferably in the form of a finely subdivided powder.

Inorganic bases which can be advantageously used in the process according to the invention are the conventional strong bases, such as, e.g., the hydroxides or the carbonates of alkali and/or alkaline-earth metals, and, preferably, sodium or potassium hydroxide and sodium or potassium carbonate, or the mixtures thereof.

The solid inorganic base is advantageously used in a molar amount comprised within the range of from 0.4 to 2 mol per mol of pyrocatechol used as the starting material.

Preferably, such molar amount is comprised within the range of from 0.5 to 1 mol per pyrocatechol mol.

The reaction is carried out in the presence of also an inert organic salt, or of a mixture of two or more inert organic solvents in different proportions.

All those organic solvents, or mixtures of organic solvents can be well used, wherein both pyrocatechol and the isopropyl halide used as the starting materials, as well as the phase-transfer catalyst used, have a highenough solubility at the reaction temperature selected, and which do not interfere with the reaction course, endangering it.

Classes of solvents which can be used in the process of the present invention comprise, e.g., the aliphatic, alycyclic or aromatic hydrocarbons, such as, e.g., n-heptane, n-octane, cyclohexane, perhydronaphthalene, toluene, xylene, and so forth, and the corresponding nitro-derivatives, such as, e.g., nitrobenzene, 2–nitroxylene, and so forth, the aliphatic and arylaliphatic alcohols, e.g., methanol, pentanol, hexanol, heptanol, benzyl alcohol, and so forth, the aliphatic ethers containing from 6 to 12 carbon atoms, such as, e.g., butyl-isopropyl-ether, methyl-pentyl-ether, butyl-ethyl-ether, heptyl-methyl-ether, di-pentyl-ether, and so forth, the aryl-($C_1$-$C_6$)-alkyl ethers, such as, e.g., anisole, hexyl-phenyl-ether, pentyl-phenyl-ether, and so forth, diaryl-ethers, e.g., di-phenyl-ether, bis-(phenyl-ethyl)-ether, etc., the cyclic ethers, e.g., dioxane, tetrahydrofuran, etc., the aliphatic ketones containing from 6 to 12 carbon atoms, such as, e.g., ethyl-pentyl-ketone, butyl-propyl-ketone, propyl-hexyl-ketone, and so forth, the aryl-($C_1$-$C_6$)-alkyl-ketones, such as, e.g., butyl-phenyl-ketone, ethyl-benzyl-ketone, benzylacetone, etc., the cyclic ketones, e.g., cycloheptanone, cyclopentanone, etc., the aliphatic and aromatic nitriles, e.g., acetonitrile, propionitrile, benzonitrile, etc., and other analogous solvents.

These solvents can be used, either individually, or as mixtures of two or more solvents in different proportions. It is also possible to use mixtures of solvents having a polarity very different from one another. In particular, the use of blends of different-polarity solvents results extremely advantageous for the separation of the products at the end of the reaction. In fact, the separation of unconverted pyrocatechol, and of the phase-transfer catalyst results particularly simple, if a solvent mixture is used, which is constituted by a low-polarity, high-boiling solvent wherein these compounds are poorly soluble at room temperature, and by a high-polarity, lower-boiling solvent, which makes it possible them to be dissolved during the course of the reaction.

In fact, at reaction end it is possible, after cooling the reaction mixture down to room temperature, and filtering off the insoluble salts, to distill off the low-boiling solvent, and separate the two organic phases which are obtained as the distillation residue. The lower organic phase substantially contains the phase-transfer catalyst, unconverted pyrocatechol, and small amounts of σ-isopropoxy-phenol, and the upper organic phase contains the desired reaction product. The lower phase can be therefore recycled as such, without undergoing any purification treatments, and σ-isopropoxy-phenol is simply recovered from the upper phase by means of classic methods, i.e., by distillation.

Polyether compounds which can be well used as the phase-transfer catalysts in the process according to the present invention are the polyethylene-glycols, having the general formula

$$HO\text{-}(CH_2\text{-}CH_2\text{-}O)_n\text{-}H$$

wherein $\underline{n}$ means an integer comprised within the range of from 2 to 200, and cyclic poly-ethers, such as crown ethers, e.g., 18-crown-6, 15-crown-5, and their dibenzo-derivatives, and cryptant substances, e.g., Kryptofix 222.

According to a preferred practical embodiment, the process of the instant invention is carried out by using a polyethylene-glycol as the phase transfer catalyst. In fact, these products offer the same performance as of the crown-ethers and of cryptants, but are, at the same time, extremely cheaper than these, do not pose any problems of noxiousness or of toxicity, and remain indefinitely active, thanks to their stability even at high temperatures.

In particular, polyethylene-glycols can be advantageously used, which have an average molecular weight which can range from 100 (n = 2–3) to 6,000 (n= comprised on the average from 150 to 200).

Very good results were anyway obtained by using as the catalyst a polyethylene-glycol having an average molecular weight comprised within the range of from 150 to 1,500.

The ratio by weight of pyrocatechol used as the starting material to the phase transfer catalyst used is preferably comprised within the range of from 0.1 to 20.

The reaction is generally carried out with stirring, at a temperature comprised within the range of from 40 to 230°C, and, preferably, of from 60 to 200°C, within a pressure range generally comprised between atmospheric pressure and 29,42 bar (30 atm), and preferably between 4,90 bar and 19,61 bar (5 and 20 atm).

Furthermore, in order to prevent an oxidation of pyrocatechol used as the starting material, and any possible degradation of the reaction product, operating under an inert gas atmosphere, typically a nitrogen atmosphere, and in the presence of small amounts of a mildly reducing agent of inorganic, or even organic, nature, such as an alkaline sulphite or hydrosulphite, an alkaline oxalate, etc., is recommended.

After a time period generally comprised within the range of from 0.5 to 20 hours, and preferably comprised within the range of from 6 to 12 hours, according to the temperature and pressure conditions under which the reaction is carried out, the desired product is separated by substantially following the above disclosed procedure, i.e., decreasing the temperature down to room value, filtering off the inorganic salts, distilling off the solvent, and then de-mixing the two organic phases, and the phase containing unconverted pyrocatechol and the phase transfer catalyst being recycled as such, and the phase containing o-isopropoxy-phenol being submitted to distillation.

The process according to the present invention supplies the desired product with extremely high values of conversion of pyrocatechol used as the starting material, and of selectivity, which can be expressed as the yield of o-isopropoxy phenol relatively to converted pyrocatechol.

The following examples, which depict in greater detail the process of the present invention, are not to be interpreted as being limitative of the purview thereof.

Example 1

To an autoclave of 1 litre of capacity, equipped with thermometer, stirrer, and heating jacket, pyrocatechol (110 g, 1 mol), isopropyl chloride at 99% (121.3 g, 1.5 mol), sodium carbonate (58.4 g, 0.55 mol), sodium hydrosulphite (1.6 g), n-decane (560 ml), isobutyl alcohol (240 ml), and polyethyleneglycol with an average molecular weight of 200 (77 g) as the catalyst, are charged.

The mixture is heated with stirring to 160°C, under a pressure value which reaches 17,65 bar (18 atm) max., also due to the development of $CO_2$ during the reaction.

The reaction mixture is kept under these conditions 10 hours long. It is then cooled down to room temperature, and is then filtered in order to remove the inorganic salts.

The filtrate is distilled in order to recover unreacted isopropyl chloride and isobutyl alcohol, which can be recycled to the next reaction. The distillation residue gets de-mixed into two phases, which are separated by cooling and subsequent decantation.

The two organic phases are analysed by gas-chromatography.

Pyrocatechol conversion results of 79%, and the yield of o-isopropoxy-phenol relatively to converted pyrocatechol results of 89%.

The upper, paraffinic, phase, containing σ-isopropoxy-phenol and reaction byproducts, is distilled, and the recovered solvent can be then recycled.

Raw o-isopropoxy-phenol is then purified by fractional distillation under vacuum.

The lower phase, containing unconverted pyrocatechol, the catalyst, a fairly large amount of o-isopropoxy-phenol and a few of paraffinic solvent, can be recycled as such to a next reaction.

Example 2

The procedure disclosed in preceding Example 1 is repeated with the only difference that as the phase transfer catalyst a polyethylene-glycol is used, which has a molecular weight of 1,540 instead of 200 (77 g).

From the gas-chromatographic analysis, is results that pyrocatechol conversion is of 80%, and the yield of o-isopropoxy-phenol relatively to converted pyrocatechol equals 87%.

Example 3

The procedure disclosed in Example 1 is repeated with the only difference that the reaction is carried

out at 120°C, with a maximum pressure of 11,76 bar (12 atm) being reached.
The gas-chromatographic analysis shows that pyrocatechol conversion was of 62%, and the yield of o-isopropoxy-phenol relatively to converted pyrocatechol was of 89%.

Example 4

The procedure disclosed in Example 1 is repeated using perhydro-naphthalene instead of n-decane.
The gas-chromatographic analysis shows a pyrocatechol conversion of 79%, and a yield of o-isopropoxy-phenol relatively to converted pyrocatechol of 88%.

Example 5

The synthesis is repeated analogously to as disclosed in Example 1, but using isobutyl alcohol as the only solvent.
During the cooling of the reaction mixture, n-decane (560 ml) is added, the inorganic salts are then filtered off, and the filtrate is distilled in order to recover isobutyl alcohol and unreacted isopropyl halide.
The distillation residue is constituted by two phases which are separated at room temperature.
The gas-chromatographic analysis of the two organic phases showed a pyrocatechol conversion of 78%, and an o-isopropoxy-phenol yield, relative to converted pyrocatechol, of 81%.

Example 6

The procedure of Example 3 is substantially repeated, with the amount of polyethylene-glycol being decreased (47 g).
From gas-chromatographic analysis, pyrocatechol conversion resulted to be of 58%, and the yield of O-isopropoxy-phenol, relatively to converted pyrocatechol, was of 81%.

Example 7

The preparation of o-isopropoxy-phenol is repeated according to a procedure substantially analogous to that of Example 3, but using a reduced-capacity (100 ml) reactor, and methyl-isobutyl-ketone (61 ml) as the only solvent, and with no addition of polyethylene-glycol.
After 15 hours of reaction, the reaction mixture, analysed by gas-chromatography, results to have the following composition:
o-isopropoxy-phenol 8, 31%
pyrocatechol 89.91%
other products 1.78%

Examples 8–9

The procedure of preceding Example 7 is repeated, respectively using, as the only solvent, anisole and nitrobenzene, in place of methyl-butyl-ketone.
In the Table I, the results of the gas-chromatographic analyses of the reaction-end mixtures are reported.

Table I

| Example No. | Solvent | O-Isopropoxy-phenol % | Pyrocatechol % | Others |
|---|---|---|---|---|
| 8 | Anisole (61 ml) | 3.25 | 96.25 | 0.50 |
| 9 | Nitrobenzene (61 ml) | 10.50 | 88.29 | 1.21 |

Examples 10–12

The procedure of Examples 7, 8 and 9 is repeated, but with the addition of a polyethylene-glycol having a molecular weight of 200 (5 g), as the catalyst.

In Table II, the results of the gas-chromatographic analyses of the reaction-end mixtures are reported.

Table II

| Example No. | Solvent | o-Isopropoxy-phenol % | Pyrocatechol % | Others |
|---|---|---|---|---|
| 10 | Methyl-isobutyl-ketone (61 ml) | 65.68 | 21.21 | 13.11 |
| 11 | Anisole (61 ml) | 60.21 | 35.01 | 4.78 |
| 12 | Nitrobenzene (61 ml) | 67.31 | 20.13 | 12.56 |

Example 13

The procedure as disclosed in Example 3 is repeated with the difference that no use is made of polyethylerne-glycol, but isobutanol is used both as the solvent, and as a substitute for polyethylene-glycol.

The gas-chromatographic analysis shows that a conversion of pyrocatechol of 25.6%, and a yield of o-isopropoxy-phenol, relative to converted pyrocatechol, of 72.6%, was obtained.

**Claims**

1. Process for the synthesis of o-isopropoxy-phenol, having formula (I):

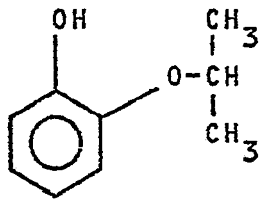

by means of the reaction of pyrocatechol, of formula (II),

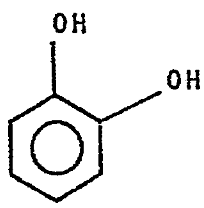

with an isopropyl halide having formula (III)

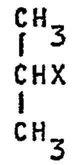

wherein X represents a halogen atom, in the presence of a solid inorganic base, and of a solid-liquid phase-transfer catalyst, inside a reaction medium constituted by one or more inert organic solvents, characterized in that the phase-transfer catalyst is a polyether selected from the polyethylene-glycols having formula

$HO-(CH_2-CH_2-O)_n-H$,

wherein n is an integer comprised within the range of from 2 to 200, the crown-ethers and the cryptants.

2. Process according to claim 1, wherein the phasetransfer catalyst is a polyethylene-glycol having a molecular weight comprised within the range of from 100 to 6,000.

3. Process according to claim 2, wherein the phasetransfer catalyst is a polyethylene-glycol having a molecular weight comprised within the range of from 150 to 1,500.

4. Process according to claim 1, wherein the phasetransfer catalyst is a crown-ether containing from 4 to 8 oxygen atoms.

5. Process according to claim 1, wherein X is a chlorine atom.

6. Process according to claim 1, wherein the solvent medium is constituted by one solvent, or a plurality of solvents mixed with one another, selected from hydrocarbons and their nitro-derivatives, alcohols, ethers, ketones and aliphatic or aromatic nitriles.

7. Process according to claim 6, wherein the solvent medium is constituted by one solvent, or a plurality of solvents mixed with one another, selected from aliphatic, alicyclic and aromatic hydrocarbons and their nitroderivatives, the aliphatic alcohols, the di-alkylketones, and the alkyl aryl-ethers.

8. Process according to claim 1, wherein the reaction is carried out with stirring, under a pressure comprised within the range of from 0,986 bar to 29,6 bar (1 to 30 atm).

9. Process according to claim 8, wherein the pressure is comprised within the range of from 5 to 20 atm.

10. Process according to claim 1, wherein the reaction is carried out at a temperature comprised within the range of from 40 to 230°C, for a time of from 0.5 to 20 hours.

11. Process according to claim 10, wherein the temperature is comprised within the range of from 60 to 200°C.

12. Process according to claim 1, wherein the molar ratio between isopropyl halide and pyrocatechol is comprised within the range of from 0.5 to 2.5.

13. Process according to claim 12, wherein the molar ratio between isopropyl halide and pyrocatechol is comprised within the range of from 0.8 to 2.

14. Process according to claim 1, wherein the solid inorganic base is selected from sodium and potassium hydroxides and carbonates.

15. Process according to claim 1, wherein the molar ratio between pyrocatechol and the solid inorganic base is comprised within the range of from 2.5 to 0.5.

16. Process according to claim 1, wherein the molar ratio between the pyrocatechol used as the starting material and the phase-transfer catalyst is comprised within the range of from 0.1 to 20.

17. Process according to claim 6, wherein the solvent medium is constituted by a mixture of inert organic solvents, one of which is a polar solvent, preferably a low-boiling alcohol, and one is a low-polarity solvent, preferably a high-boiling hydrocarbon.

**Patentansprüche**

1. Verfahren zur Herstellung von o-Isopropoxyphenol mit der Formel (I):

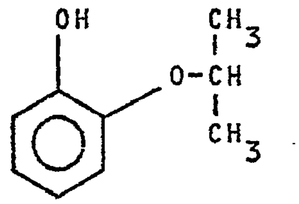

I

durch Umsetzung von Brenzcatechin der Formel (II):

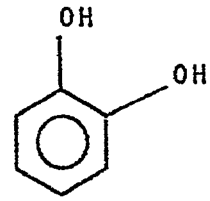

(II)

mit einem Isopropylhalogenid mit der Formel (III):

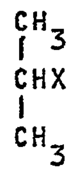

(III)

worin X ein Halogenatom darstellt, in Gegenwart einer festen anorganischen Base und eines Fest-Flüssig-Phasentransferkatalysators in einem aus einem oder aus mehreren inerten organischen Lösungsmitteln gebildeten Reaktionsmedium, dadurch gekennzeichnet, daß der Phasentransferkatalysator ein unter den Polyethylenglykolen der Formel

$HO-(CH_2-CH_2-O)_n-H$,

worin n eine ganze Zahl im Bereich von 2 bis 200 darstellt, den Kronenethern und den Kryptanden ausgewählter Polyether ist.

2. Verfahren nach Anspruch 1, worin der Phasentransferkatalysator ein Polyethylenglykol mit einem Molekulargewicht im Bereich von 100 bis 6.000 ist.

3. Verfahren nach Anspruch 2, worin der Phasentransferkatalysator ein Polyethylenglykol mit einem Molekulargewicht im Bereich von 150 bis 1.500 ist.

4. Verfahren nach Anspruch 1, worin der Phasentransferkatalysator ein 4 bis 8 Sauerstoffatome enthaltender Kronenether ist.

5. Verfahren nach Anspruch 1, worin X ein Chloratom bedeutet.

6. Verfahren nach Anspruch 1, worin das Lösungsmittelmedium aus einem Lösungsmittel oder einer Mehrzahl von miteinander vermischten Lösungsmitteln, ausgewählt unter Kohlenwasserstoffen und ihren Nitroderivaten, Alkoholen, Ethern, Ketonen und aliphatischen oder aromatischen Nitrilen, gebildet ist.

7. Verfahren nach Anspruch 6, worin das Lösungsmittelmedium aus einem Lösungsmittel oder aus einer Mehrzahl von miteinander vermischten Lösungsmitteln, ausgewählt unter aliphatischen, alicyclischen und aromatischen Kohlenwasserstoffen und ihren Nitroderivaten, den aliphatischen Alkoholen, den Dialkylketonen und den Alkylarylethern, gebildet ist.

8. Verfahren nach Anspruch 1, worin die Umsetzung unter Rühren und unter einem Druck im Bereich von 0,986 bar bis 29,6 bar (1 bis 30 at) ausgeführt wird.

9. Verfahren nach Anspruch 8, worin der Druck im Bereich von 5 bis 20 at gehalten wird.

10. Verfahren nach Anspruch 1, worin die Umsetzung bei einer Temperatur im Bereich von 40 bis 230°C während einer Zeit von 0,5 bis 20 Stunden ausgeführt wird.

11. Verfahren nach Anspruch 10, worin die Temperatur im Bereich von 60 bis 200°C gehalten wird.

12. Verfahren nach Anspruch 1, worin das Molverhältnis zwischen Isopropylhalogenid und Brenzkatechin im Bereich von 0,5 bis 2,5 gehalten wird.

13. Verfahren nach Anspruch 12, worin das Molverhältnis zwischen Isopropylhalogenid und Brenzkatechin im Bereich von 0,8 bis 2 gehalten wird.

14. Verfahren nach Anspruch 1, worin die feste anorganische Base unter Natrium- und Kaliumhydroxiden und -carbonaten ausgewählt wird.

15. Verfahren nach Anspruch 1, worin das Molverhältnis zwischen Brenzkatechin und fester anorganischer Base im Bereich von 2,5 bis 0,5 gehalten wird.

16. Verfahren nach Anspruch 1, worin das Molverhältnis zwischen dem als Ausgangsmaterial verwendeten Brenzkatechin und dem Phasentransferkatalysator im Bereich von 0,1 bis 20 gehalten wird.

17. Verfahren nach Anspruch 6, worin das Lösungsmittel aus einem Gemisch aus inerten organischen Lösungsmitteln gebildet ist, von denen eines ein polares Lösungsmittel, vorzugsweise ein niedrig siedender Alkohol ist und eines ein Lösungsmittel mit niedriger Polarität ist, vorzugsweise ein hochsiedender Kohlenwasserstoff.

**Revendications**

1. Procédé pour la synthèse de l'o-isopropoxyphénol de formule (I):

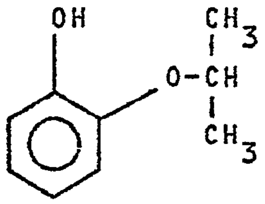

(I)

par réaction du pyrocatéchol de formule (II):

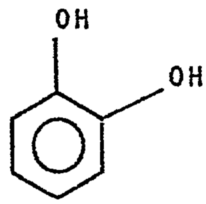

(II)

avec un halogénure d'isopropyle de formule (III)

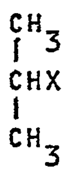

dans laquelle X représente un atome d'halogène, en présence d'une base minérale solide et d'un catalyseur de transfert de phase solide-liquide, au sein d'un milieu réactionnel constitué par un ou plusieurs solvant(s) organique(s) inerte(s), caractérisé en ce que le catalyseur de transfert de phase est un polyéther choisi parmi les polyéthylène-glycols de formule

$HO-(CH_2-CH_2-O)_n-H$,

dans laquelle n est un nombre entier compris dans l'intervalle allant de 2 à 200, les éthers-couronnes et les cryptants.

2. Procédé selon la revendication 1, dans lequel le catalyseur de transfert de phase est un polyéthylèneglycol ayant une masse moléculaire située dans l'intervalle allant de 100 à 6000.

3. Procédé selon la revendication 2, dans lequel le catalyseur de transfert de phase est un polyéthylèneglycol ayant une masse moléculaire située dans l'intervalle allant de 150 à 1500.

4. Procédé selon la revendication 1, dans lequel le catalyseur de transfert de phase est un éther-couronne contenant de 4 à 8 atomes d'oxygène.

5. Procédé selon la revendication 1, dans lequel X est un atome de chlore.

6. Procédé selon la revendication 1, dans lequel le milieu solvant est constitué par un solvant ou plusieurs solvants mélangés, choisi(s) parmi les hydrocarbures et leurs dérivés nitro, les alcools, les éthers, les cétones et les nitriles aliphatiques ou aromatiques.

7. Procédé selon la revendication 6, dans lequel le milieu solvant est constitué par un solvant ou plusieurs solvants mélangés, choisi(s) parmi les hydrocarbures aliphatiques, alicycliques et aromatiques et leurs dérivés nitro, les alcools aliphatiques, les dialkylcétones et les éthers d'alkyle et d'aryle.

8. Procédé selon la revendication 1, dans lequel la réaction est effectuée sous agitation, sous une pression située dans l'intervalle allant de 0,986 bar à 29,6 bars (1 à 30 atm).

9. Procédé selon la revendication 8, dans lequel la pression est située dans l'intervalle allant de 5 à 20 atm.

10. Procédé selon la revendication 1, dans lequel la réaction est effectuée à une température située dans l'intervalle allant de 40 à 230°C, pendant une durée de 0,5 à 20 heures.

11. Procédé selon la revendication 10, dans lequel la température est située dans l'intervalle allant de 60 à 200°C.

12. Procédé selon la revendication 1, dans lequel le rapport molaire entre l'halogénure d'isopropyle et le pyrocatéchol est situé dans l'intervalle allant de 0,5 à 2,5.

13. Procédé selon la revendication 12, dans lequel le rapport molaire entre l'halogénure d'isopropyle et le pyrocatéchol est situé dans l'intervalle allant de 0,8 à 2.

14. Procédé selon la revendication 1, dans lequel la base minérale solide est choisie parmi les hydroxydes et carbonates de sodium et de potassium.

15. Procédé selon la revendication 1, dans lequel le rapport molaire entre le pyrocatéchol et la base minérale solide est situé dans l'intervalle allant de 2,5 à 0,5.

16. Procédé selon la revendication 1, dans lequel le rapport molaire entre le pyrocatéchol utilisé comme matériau de départ et le catalyseur de transfert de phase est situé dans l'intervalle allant de 0,1 à 20.

17. Procédé selon la revendication 6, dans lequel le milieu solvant est constitué par un mélange de solvants organiques inertes, dont l'un est un solvant polaire, de préférence un alcool à bas point d'ébullition, et un autre est un solvant faiblement polaire, de préférence un hydrocarbure à point d'ébullition élevé.